# EUROPEAN PATENT APPLICATION

(11) **EP 1 645 875 A1**
(43) Date of publication of application: **12.04.2006**
(21) Application number: 04024054.1
(22) Date of filing: 08.10.2004
(51) Int. Cl.: G01N 33/48, A61K 39/395

(54) **Diagnosis and therapy of cell proliferative disorders characterized by resistance to TRAIL induced apoptosis**

(71) Applicant: Deutsches Krebsforschungszentrum Stiftung des öffentlichen Rechts, 69120 Heidelberg (DE)
(72) Inventor: Wolf, Stephan, 66969 Lemberg (DE); Joos, Stefan, 69120 Heidelberg (DE); Mertens, Daniel, 69168 Schriesheim (DE); Pscherer, Armin, 69121 Heidelberg (DE); Lichter, Peter, 69251 Gaiberg (DE)
(74) Representative: Schüssler, Andrea

(57) **Abstract**

Described are methods and compounds for diagnosis and therapy of subsets of cell proliferative disorders which are characterized by resistance to TRAIL induced apoptosis. Examples of such diseases are B-cell chronic lymphocytic leukemia (CLL), mantle cell lymphoma (MCL) and prostate cancer. Furthermore, methods for identifying drugs which are suitable for treatment of such diseases are described.

## Description

The present invention relates to the diagnosis and therapy of cell proliferative disorders which are characterized by resistance to TRAIL induced apoptosis. Examples of such diseases are subsets of B-cell chronic lymphocytic leukemia (CLL), mantle cell lymphoma (MCL) and prostate cancer. Furthermore, the present invention relates to methods for identifying drugs which are suitable for treatment of such diseases.

B-cell chronic lymphocytic leukemia (CLL) represents one of the most common hematological malignancies in western countries. The disease is associated with an accumulation of mature, non cycling CD5/CD19-positive B lymphocytes (Rozman and Montserrat, *N*. *Engl. J. Med.* **333**(16), 1052-1057 (1995)). In CLL, the accumulation of malignant cells results from impairment of apoptosis rather than excessive cellular proliferation that is postulated for the closely related mantle cell lymphoma (MCL). Recently, it has been reported that CLL cells are resistant to *tumor necrosis factor-related apoptosis inducing ligand* (TRAIL) induced apoptosis upstream of caspase-8 activation (MacFarlane et *al., Oncogene* **21**(44), 6809-6818 (2002)). No mutation of any gene involved in the TRAIL induced apoptotic pathway has been reported in hematological malignancies so far.

The human neoplasms CLL and MCL are B-cell non-Hodgkin lymphomas of low and intermediate grade, respectively. CLL is characterized by the accumulation of mature, G0 resting B-cells in peripheral blood (PB), bone marrow, spleen and lymph nodes (Dameshek, Blood **29**(4), 566-584 (1967)). Standard treatments for CLL include the alcylating agent chlorambucil (CLB) and the nucleoside analog fludarabine (FLU, F-ara-AMP) (Dighiero and Binet, *N. Engl. J. Med.* **343**(24) 1799-1801 (2000); Rai et *al*., *N. Engl. J. Med.* **343**(24), 1750-1757 (2000). Both agents are promoting apoptosis via activation of caspases (Begleiter et al., *Leuk. Lymphoma.* **23**(3-4), 187-201 (1996)). CLL and MCL have a closely related pattern of genomic abnormalities with frequent loss of material in 13q14.3, 11q22.3-q23.1, 6q21-q23 and 17p13, whereas loss of material in chromosomal band 8p21 is recurrently observed only in MCL. Within this chromosomal region the TRAIL-induced death receptors TNFRSF10A and TNFRSF10B are localized ⁽MacFarlane et *al., J. Biol. Chem.* **272**(41), 25417-25420 (1997).

Based on its induction of cell death in various tumor cell lines and its lack of toxicity to most normal cells, TRAIL has recently emerged as a novel potential anti-cancer agent (Ashkenazi and Dixit, *Curr. Opin. Cell. Biol.* **11**(2), 255-260 (1999); Walczak_et *al., Nat Med.* **5**(2), 157-163 (1999)). TRAIL interacts with at least four membrane-bound receptors: TNFRSF10A (DR4), TNFRSF10B (DR5, TRICK2), TNFRSF10C (TRID, DcRl, LIT) and TNFRSF10D (DcR2, TRUNDD) (Ashkenazi and Dixit, *Science* **281**(5381), 1305-1308 (1998)). Both TNFRSF10A and TNFRSF10B contain a conserved death domain. Binding of TRAIL to its receptors results in trimerization of the receptors and clustering of their intracellular death domains (DD). This leads to the formation of death-inducing signaling complexes (DISC) (Boldin et al., *Cell* **85**(6), 803-815 (1996); Muzio et *al., Cell* **85**(6), 817-827 (1996)) followed by the recruitment of the adaptor molecule Fas-associated death receptor (FADD) and subsequent binding and activation of caspase-8 and caspase-10. Recently, CLL cells were shown to be resistant to TRAIL induced apoptosis (MacFarlane et *al., Oncogene* **21**(44), 6809-6818 (2002)). This inhibition of apoptosis has to be upstream of caspase-8 activation since little or no active caspase-8 protein is detectable in TRAIL treated CLL cells. However, mutation analysis of the entire TNFRSF10B gene and the death domain of TNFRSF10A revealed no disease correlated aberrations in different tumor types so far. In siRNA experiments, it was recently shown that TNFRSF10A mediates the majority of TRAIL induced apoptosis in HeLa cells (Aza-Blanc *et al., Mol. Cell* 12(3), 627-637 (2003)). Since, however, the molecular mechanism underlying the resistance of cancer cells like B-CLL cells to TRAIL induced apoptosis has not been revealed, a specific therapy of malignancies which show resistance to TRAIL induced apoptosis is so far not available

Thus, the technical problem underlying the present invention is to provide means for therapy and diagnosis of a subset of malignancies characterized by apoptosis resistance.

The solution to said technical problem is achieved by providing the embodiments characterised in the claims. During the experiments resulting in the present invention it could be shown that a rare variant of the *tumor necrosis factor-related apoptosis inducing ligand* receptor 1 gene (TNFRSF10A), which leads to an amino acid substitution Glu228Ala in the cysteine-rich TRAIL/TNFRSF10A interaction domain of TNFRSF10A, is associated with CLL,MCL and prostate cancer. In order to functionally assess the pathogenic role of this gene polymorphism, altered cDNA constructs were synthesized producing TRAIL-ligand peptides compatible with this rare variant of TNFRSF10A and these peptides were applied to the respective cells. It could be demonstrated that they are capable of reconstituting caspase-8 dependent apoptosis induction in CLL and prostate cancer cells carrying the Glu228Ala variant of TNFRSF10A, which were resistant to wildtype- (WT-) TRAIL induced apoptosis. These findings contribute to the elucidation of the pathomechanism of CLL, MCL and prostate cancer and provide a basis for the design of new drugs in the therapy of cancer patients, e.g., CLL patients carrying the rare Ala228 variant of TNFRSF10A.

Accordingly, in a first aspect, the present invention relates to a method for diagnosing subsets of cell proliferative disorders, characterized by resistance to TRAIL induced apoptosis or a predisposition for such disorder, comprising determining the biological activity and/or level of a TRAIL receptor in a sample from a patient wherein a reduced or eliminated biological activity or level of said TRAIL receptor is indicative of such disorder or predisposition.

The present invention also provides a method for detecting a cell proliferative disorder associated with a metastasizing tumor which comprises contacting a sample suspected to contain a specific TRAIL receptor variant with a reagent which allows analysing amino acid sequence or nucleic acid sequence of the corresponding gene. When the target is the gene or mRNA, the reagent is typically a nucleic acid probe or a primer for PCR. The person skilled in the art is in a position to design suitable nucleic acid probes based on the information as regards the nucleotide sequence of TRAIL receptors [Real-time PCR assay for quantitative mismatch detection. Biotechniqes 2003, Shively L, Chang L, LeBon JM, Liu Q, Riggs AD, Singer-Sam J.; Quantitative real-time RT-PCR using hybridization probes and imported standard curves for cytokine gene expression analysis. Biotechniques 2002, Kuhne BS, Oschmann P.]. When the target is the protein, the reagent is typically an antibody probe. The term "antibody", preferably, relates to antibodies which consist essentially of pooled monoclonal antibodies with different epitopic specificities, as well as distinct monclonal antibody preparations. Monoclonal antibodies are made from an antigen containing fragments of the TRAIL receptor protein by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments, scFv, etc.) which are capable of specifically binding to protein. The target cellular component, i.e. the TRAIL receptor or the receptor encoding mRNA, e.g., in biological fluids or tissues, may be detected directly in situ or it may be isolated from other cell components by common methods known to those skilled in the art before contacting with a probe. Detection methods include Northern blot analysis, RNase protection, in situ methods, PCR, LCR, SDA, sequencing, immunoassays and other detection assays that are known to those skilled in the art.

Useful tissue samples includes cells derived from peripheral blood or sputum.

The probes can be detectably labeled, for example, with a radioisotope, a bioluminescent compound, a chemiluminescent compound, a fluorescent compound, a metal chelate, or an enzyme.

TRAIL receptor expression in tissues can be studied with classical immunohistological methods (Jalkanen et al., J. Cell. Biol. 101 (1985), 976-985; Jalkanen et al., J. Cell. Biol. 105 (1987), 3087-3096). Other antibody based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I) , carbon (¹⁴C) , sulfur (³⁵*S*) , tritium (³H) , indium (¹¹²In), and technetium (⁹⁹mTc), and fluorescent labels, such as fluorescein and rhodamine, and biotin. In addition to assaying TRAIL receptor levels in a biological sample, TRAIL receptors can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment which has been labeled with an appropriate detectable imaging moiety, such as a radioisotope (for example, ¹³¹I, ¹¹²In, ⁹⁹mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982)).

For evaluating whether the concentration or activity of the TRAIL receptor is decreased or whether the amino acid- or nucleic acid sequence contains a mutation interfering with biological activity, thus being indicative for a disease characterized by resistance to TRAIL induced apoptosis, the determined concentration/activity/sequence is compared with the concentration/activity/sequence in a normal tissue.

In a preferred embodiment of the diagnostic method of the present invention, possible targets are all receptors of TRAIL, preferably TNFRSF10A.

In a more preferred embodiment, TNFRSF10A is characterized by a variation within its extracellular domain resulting in a reduced or eliminated ligand (TRAIL) binding and reduced death signaling.

In an even more preferred embodiment of the diagnostic method of the present invention, the TRAIL receptor is TNFRSF10A containing the amino acid substitution Glu228Ala.

The method of the present invention is particularly useful for the diagnosis of subsets of CLL, MCL or prostate carcinoma with a reduced sensitivity to TRAIL induced apoptosis.

In a further aspect, the present invention relates to a polynucleotide encoding TNFRSF10A which is characterized by a rare nucleotide composition within its extracellular domain encoding region, resulting in a reduced or eliminated TRAIL binding of the resulting protein. Preferably, said mutation is A683C.

The present invention also relates to a polymorphism of TNFRSF10A encoded by a polynucleotide as described above or a fragment thereof which are, e.g., useful in screening method for compounds, e.g., modified ligands, resulting in reactivation of the modified TRAIL receptor. Preferably, the mutated TRAIL or fragment thereof are recombinantly produced by cultivating a host cell transformed with an expression vector described below under conditions allowing the synthesis of the peptide and the peptide is subsequently isolated from the cultivated cells and/or the culture medium. Isolation and purification of the recombinantly produced proteins may be carried out by conventional means including preparative chromatography and affinity and immunological separations involving affinity chromatography with monoclonal or polyclonal antibodies.

For recombinant production of the mutated TRAIL peptides thereof, the DNA sequences encoding the mutated TRAIL or fragment thereof are inserted in a recombinant vector, e.g. an expression vector. Preferably, the vectors are plasmids, cosmids, viruses, bacteriophages and other vectors usually used in the field of genetic engineering. Vectors suitable for use in the present invention include, but are not limited to the T7-based expression vector for expression in bacteria, the pMSXND expression vector for expression in mammalian cells and baculovirus-derived vectors for expression in insect cells. Preferably, the DNA sequences are operatively linked to the regulatory elements in the recombinant vector that guarantee the transcription and synthesis of an RNA in prokaryotic and/or eukaryotic cells that can be translated. The nucleotide sequence to be transcribed can be operably linked to a promoter like a T7, metallothionein I or polyhedrin promoter. Peptides can also be produced in a cell free in vitro translation system, using plasmid DNA or a specific PCR template containing the required regulatory sequences for translation.

In a further aspect, the present invention relates to a polynucleotide encoding a modified TRAIL or TRAIL-fragment, capable of binding to a mutated TRAIL receptor as described above.

In a preferred embodiment, said polynucleotide encodes a TRAIL protein wherein the amino acid residue Asn at position 199 of the modified TRAIL is substituted by a different amino acid residue, preferably by Glu or Arg.

The present invention also relates to a modified TRAIL protein encoded by a polynucleotide as described above. Such a modified TRAIL (or the polynucleotide encoding it) is useful for therapy as described, e.g., in the examples of the present invention.

Preferred recombinant vectors containing, e.g., a modified TRAIL protein encoding DNA as described above, useful for gene therapy are viral vectors, e.g. adenovirus, AAV, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines containing plasmids encoding all of the structural genes of the retrovirus under the control of regulatory sequences within the LTR. Suitable helper cell lines are well known to those skilled in the art. Said vectors additionally can contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors Further suitable vectors and methods for in vitro- or in vivo-gene therapy are described in the literature and are known to the persons skilled in the art; see, e.g., WO 94/29469 or WO 97/00957. In order to achieve expression only in the target organ, the nucleic acids can also be operably linked to a tissue specific promoter and used for gene therapy.

In a further embodiment, the present invention relates to recombinant host cells transiently or stably containing the nucleic acid molecules or vectors as described above. A host cell is understood to be an organism that is capable to take up *in vitro* recombinant DNA and, if the case may be, to synthesize the proteins encoded by the nucleic acid molecules as described above. Preferably, these cells are prokaryotic or eukaryotic cells, for example mammalian cells, bacterial cells, insect cells or yeast cells. The host cells of the invention are preferably characterized by the fact that the introduced nucleic acid molecules either are heterologous with regard to the transformed cell, i.e. that they do not naturally occur in these cells, or are localized at a place in the genome different from that of the corresponding naturally occurring sequences.

In a further aspect, the present invention relates to a method of identifying an agonist/activator of the mutated TRAIL receptor as defined above, comprising the following steps:
(a) preparing a candidate compound;
(b) contacting a cell which expresses said TRAIL receptor on its surface with said candidate compound; and
(c) determining whether said candidate compound activates said TRAIL receptor.

Steps (a) and (b) can be carried out according to routine methods and step (c) can be performed in line with the instructions given, e.g., in Examples 1 and 4, below.

Candidate compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, candidate compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. See Lam, *Anticancer Drug Des.* 12, 145, 1997.

Methods for the synthesis of molecular libraries are well known in the art (see, for example, DeWitt *et al., Proc. Natl. Acad. Sci. U.S.A. 90,* 6909, 1993; Erb *et al. Proc. Natl. Acad. Sci. U.S.A. 91,* 11422, *1994;* Zuckermann *et al., J. Med. Chem. 37,* 2678, 1994; Cho *et al., Science 261*, 1303, 1993; Carell *et al., Angew. Chem. Int. Ed. Engl.* 33, 2059, 1994; Carell *et al., Angew. Chem. Int. Ed. Engl. 33,* 2061; Gallop *et al., J. Med. Chem.* 37, 1233, 1994). Libraries of compounds can be presented in solution *(see, e.g.,* Houghten, *Biotechniques* 13, 412-421, 1992), or on beads (Lam, Nature 354, 82-84, 1991), chips (Fodor, *Nature* 364, 555-556, 1993), bacteria or spores (Ladner, U.S. Patent 5,223,409), plasmids (Cull et *al., Proc. Natl. Acad. Sci. U.S.A.* 89, 1865-1869, 1992), or phage (Scott & *SmithScience* 249, 386-390, 1990; Devlin, *Science* 249, 404-406, 1990); Cwirla et *al., Proc. Natl. Acad*. *Sci.* 97, 6378-6382, 1990; Felici, *J. Mol. Biol.* 222, 301-310, 1991; and Ladner, U.S. Patent 5,223,409).

Candidate compounds can be screened for the ability to bind to a modified TRAIL receptor or to activate the modified TRAIL receptor using high throughput screening. Using high throughput screening, many discrete compounds can be tested in parallel so that large numbers of candidate compounds can be quickly screened. The most widely established techniques utilize 96-well mierotiter plates. The wells of the mierotiter plates typically require assay volumes that range from 50 to 500. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad. Sci. USA. 19,* 1614-18 (1994). The cells are placed under agarose in petri dishes, then beads that carry combinatorial compounds are placed on the surface of the agarose. Active compounds can be visualized as dark pigment areas because, as the compounds diffuse locally into the gel matrix, the active compounds cause the cells to change colors.

Another example of a free format assay is described by Chelsky,
"Strategies for Screening Combinatorial Libraries: Novel and Traditional Approaches," reported at the First Annual Conference of The Society for Biomolecular Screening in Philadephia, Pa. (Nov. 7-10, 1995). A simple homogenous enzyme assay for carbonic anhydrase was placed inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change. Yet another example is described by Salmon *et al., Molecular Diversity 2, 57-63 (1996).* In this example, combinatorial libraries were screened for compounds that had cytotoxic effects an cancer cells growing in agar.

Another high throughput screening method is described in Beutel et *al., U.S.* Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

For binding assays, the test compound is preferably a small molecule which binds and occupies the active site of the modified TRAIL receptor. Examples of such small molecules include, but are not limited to, small peptides or peptide-like molecules. Potential ligands which bind to a polypeptide of the invention include, but are not limited to, the natural modified ligands of TRAIL-R, ligand-like proteins and analogues or derivatives thereof.

In binding assays, the candidate compound can comprise a detectable label, such as a fluorescent, radioisotopic, chemiluminescent, or enzymatic label, such as horseradish peroxidase, alkaline phosphatase, or luciferase.

Detection of a candidate compound which is bound to the modified TRAIL receptor can then be accomplished, for example, by direct counting of radioemmission, by scintillation counting, or by determining conversion of an appropriate substrate to a detectable product.

Alternatively, binding of a test compound to a modified TRAIL receptor-like polypeptide can be determined without labeling. For example, a microphysiometer can be used to detect binding of a candidate compound with a modified TRAIL receptor. A microphysiometer (e.g., Cytosensor™) is an analytical instrument that measures the rate at which a cell acidifies its environment using a light-addressable potentiometric sensor (LAPS). Changes in this acidification rate can be used as an indicator of the interaction between a candidate compound and a modified TRAIL receptor (McConnell et *al., Seience 257,* 1906-1912, 1992).

Determining the ability of a candidate compound to bind to a modified TRAIL receptor also can be accomplished using a technology such as real-time Bimolecular Interaction Analysis (BIA) (Sjolander & Urbaniczky, *Anal. Chem. 63,* 2338-2345, 1991, and Szabo et *al., Curr. Opin. Struct. Biol. 5,* 699-705, 1995). BIA is a technology for studying biospecific interactanns in real time, without labeling any of the interactants (e.g., BIAcore™). Changes in the optical phenomenon surface plasmon resonance (SPR) can be used as an indication of real-time reactions between biological molecules.

In yet another aspect of the invention, a modified TRAIL receptor can be used as a "bait protein" in a two-hybrid assay or three-hybrid assay (see, e.g., *U.S*. Patent 5,283,317; Zervos et al., *Cell* 72, 223-232, 1993; Madura *et al., J. Biol. Chem.* 30 268, 12046-12054, 1993; Bartel et al., *Biotechniques 14,* 920-924, 1993; Iwabuchi et *al., Oncogene 8,* 1693-1696, 1993; and Brent W094/10300), to identify other proteins which bind to or interact with the modified TRAIL receptor and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a modified TRAIL receptor can be fused to a polynucleotide encoding the DNA binding domain of a known transcription factor (e.g., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the modified TRAIL receptor.

It may be desirable to immobilize either the modified TRAIL receptor (or polynucleotide) or the candidate compound to facilitate separation of bound from unbound forms of one or both of the interactants, as well as to accommodate automation of the assay. Thus, either the receptor (or polynucleotide) or the candidate compound can be bound to a solid support. Suitable solid supports include glass or plastic slides, tissue culture plates, microtiter wells, tubes, silicon chips, or particles such as beads. Any method known in the art can be used to attach the modified TRAIL receptor (or polynucleotide) or candidate compound to a solid support, including use of covalent and non-covalent linkages, passive absorption, or pairs of binding moieties attached respectively to the polypeptide (or polynucleotide) or candidate compound and the solid support. Candidate compounds are preferably bound to the solid support in an array, so that the location of individual candidate compounds can be tracked. Binding of a candidate compound to a receptor (or polynucleotide) can be accomplished in any vessel suitable for containing the reactants. Examples of such vessels include microtiter plates, test tubes, and microcentrifuge tubes.

Moreover, the modified TRAIL receptor can be a fusion protein comprising a domain that allows the receptor to be bound to a solid support. For example, glutathione-S-transferase fusion proteins can be adsorbed onto glutathione sepharose or glutathione derivatized microtiter plates, which are then combined with the candidate compound or the candidate compound and the non-adsorbed receptor. The mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a modified TRAIL receptor (or polynucleotide) or a candidate compound can be immobilized utilizing conjugation of biotin and streptavidin. Biotinylated lipoxin receptor polypeptides (or polynucleotides) or candidate compounds can be prepared from biotin-NHS(N-hydroxysuccinimide) using techniques well known in the art (e.g., a biotinylation kit, Pierce Chemicals, Rockford, III.) and immobilized in the wells of streptavidin-coated 96 well plates. Alternatively, antibodies which specifically bind to a modified TRAIL receptor, polynucleotide, or a candidate compound, but which do not interfere with a desired binding site, such as the active site of the receptor, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the modified TRAIL receptor or candidate compound, enzyme-linked assays which rely an detecting an activity of the receptor, and SDS gel electrophoresis under non-reducing conditions. Screening for candidate compounds which bind to a receptor or polynucleotide also can be carried out in an intact cell.

Any cell which comprises a modified TRAIL receptor or polynucleotide can be used in a cell-based assay system. A modified TRAIL receptor can be naturally occurring in the cell or can be introduced using techniques such as those described above. Binding of the candidate compound to a receptor polypeptide or polynucleotide is determined as described above.

Candidate compounds can also be tested for the ability to increase signal transduction mediated by the TRAIL receptor, e.g., by determining apoptosis as described in the examples. In addition, functional assays include the use of cells which express the G-protein coupled receptor (for example, transfected CHO cells) in a system which measures extracellular pH changes caused by receptor activation *(see, e.g., Science 246, 181-296, 1989).* For example, candidate compounds may be contacted with a cell which expresses the modified receptor polypeptide and a second messenger response, e.g., signal transduction or pH changes, can be measured to determine whether the potential compound activates or inhibits the receptor. Functional assays can be conducted after contacting a purified modified TRAIL receptor, a cell membrane preparation, or an intact cell with a candidate compound.

Another screening technique involves expressing the G-protein coupled modified receptor in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Finally, candidate compounds which increase TRAIL receptor gene expression can be identified. A TRAIL receptor encoding polynucleotide is contacted with a candidate compound, and the expression of an RNA or polypeptide product is determined. The level of expression of appropriate mRNA or polypeptide in the presence of the candidate compound is compared to the level of expression of mRNA or polypeptide in the absence of the candidate compound. The candidate compound can then be identified as an activator of expression based an this comparison. For example, when expression of mRNA or polypeptide is greater in the presence of the candidate compound than in its absence, the candidate compound is identified as a stimulator or enhancer of the mRNA or polypeptide expression.

The level of expression in the cells can be determined by methods well known in the art for detecting mRNA or polypeptide. Either qualitative or quantitative methods can be used. The presence of polypeptide products of the receptor can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into a receptor polypeptide. Such screening can be carried out either in a cell-free assay system or in an intact cell. Any cell which expresses a TRAIL receptor encoding polynucleotide can be used in a cell-based assay system. The TRAIL receptor encoding polynucleotide can be naturally occurring in the cell or can be introduced using techniques such as these described above. Either a primary culture or an established cell line, such as CHO or human embryonic kidney 293 cells, can be used.

In a preferred embodiment of the screening method of the present invention, said candidate compound is a modified TRAIL, e.g., a modified TRAIL produced by random mutagenesis, preferably said modified TRAIL contains a mutation within the TRAIL/ TNFRSF10A interaction site resulting, preferably, in an amino acid substitution Asn199Glu or Asn199Arg.

The present invention also relates to a pharmaceutical composition containing a modified TRAIL encoding polynucleotide as described above, an expression vector containing said polynucleotide, a modified TRAIL or an agonist/activator identified by a screening method of the present invention. For administration, the above described compounds are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the proliferative disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the proliferative disease, general health and other drugs being administered concurrently.

The delivery of the above described polynucleotides can be achieved by direct application or, preferably, by using one of the recombinant expression vectors described above or a colloidal dispersion system. These polynucleotides can also be administered directly to the target site, e.g., by ballistic delivery, as a colloidal dispersion system or by catheter to a site in artery. The colloidal dispersion systems which can be used for delivery of the above polynucleotides include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired polynucleotide. Organ-specific or cell-specific liposomes can be used in order to achieve delivery only to the desired tumour. The targeting of liposomes can be carried out by the person skilled in the art by applying commonly known methods. This targeting includes passive targeting (utilising the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tissues via specific cell-surface ligands. The modified TRAIL proteins or other agonist proteins can also be delivered using the above described systems, preferably by using a liposome.

The present invention also relates to the use of the compounds described above for the preparation of a pharmaceutical composition for reconstituting TRAIL induced apoptosis in TRAIL insensitive cells, e.g., B cells, preferably for treating CLL, MCL or prostate carcinoma.

Finally, the present invention provides a method for the production of a pharmaceutical composition comprising a screening method of the present invention and furthermore mixing the agonist/activator obtained by such screening method with a pharmaceutically acceptable carrier.

### Brief description of the drawings

### Figure 1: Status of TNFRSF10A 683 A/C polymorphism in CLL-, MCL-patients and B-cell lines, peripheral blood cells from healthy controls and the estimated allele frequency from NCBI refSNP ID rs20576

683A/C heterozygosity and exclusive expression of TNFRSF10A 683C is clearly enhanced in CLL and MCL as compared to healthy controls.

### Figure 2: Recombinant, mutagenized TRAIL proteins (TLRPs) to restore obstructed apoptosis in cell lines expressing the TNFRSF10A Ala228 variant

**(a)** Crystal structure of the TRAIL/TNFRSF10B complex. Amino acid residue Glu124 of TNFRSF10B correlates with Glu/Ala228 of TNFRSF10A.
(**b**) Simplified scheme of the interaction of TRAIL/TRAILR complex formation. Mutagenized TLRPs may restore detained TRAIL/TNFRSF10A interaction and subsequent apoptosis induction in TNFRSF10A 683A/A and 683A/C cells.
(**c**) Apoptosis rates of the B-cell lines GRANTA-519, EHEB and JVM-2 treated with WT-TRAIL, TLRP-1s, TLRP-21 and protein elution buffer (mock) as negative control. A photometric immunoassay for the quantitative in vitro determination of cytoplasmic histone-associated DNA fragments after induced cell death was used.
(**d**) Rate of caspase-8 dependent apoptosis in the TNFRSF10A 683A/A expressing cell line JVM-2, treated with recombinant TLRP-1s and TLRP-21 compared to WT-TRAIL, mock and etoposide. In three independent experiments caspase-8 activation is clearly enhanced in TLRP treated JVM-2 cells as compared to cells treated with WT-TRAIL. In one experiment cells were incubated in 1% serum to increase their sensitivity to TRAIL/TLRP induced apoptosis. TLRP peptides in these experiments were produced using a cell free yeast expression assay with PCR fragments as templates.

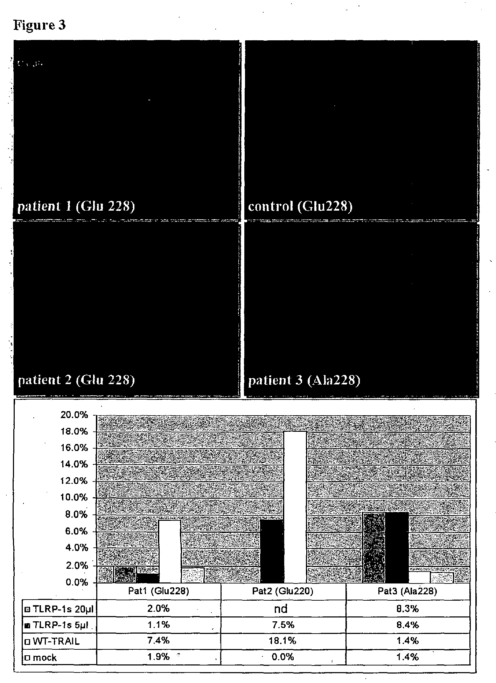

### Figure 3: Apoptosis rates in B-cells from CLL patients and healthy control, expressing TNFRSF10A Ala228 and Glu228 variants, respectively, after induction with TLRP-1s

TLRP-1s induced apoptotic cells were detected using a caspase-8 detection assay. 200-400 cells were scored in each experiment. Induction of apoptosis is enhanced in the Ala228 patient when treated with TLRP-1s as compared to WT-TRAIL. Glu228 patients exhibited an alteration in TRAIL/TLRP response.

The present invention is explained by the following examples.

### Example 1: Materials and Methods

### (A) Apoptosis and cell death detection assays

Apoptosis rates in the cell lines were measured based on the ELISA plus Apoptosis kit (Roche, Mannheim, Germany) or the Carboxyfluorescein Caspase-8 and Caspase-3 Detection Kit (Biocharta, Carlsbad, California) following the manufacturers recommendations. Wild type TRAIL (Alexis, Lausen, Switzerland) was used for apoptosis induction following the manufacturers recommendations .

### (B) Protein expression and purification

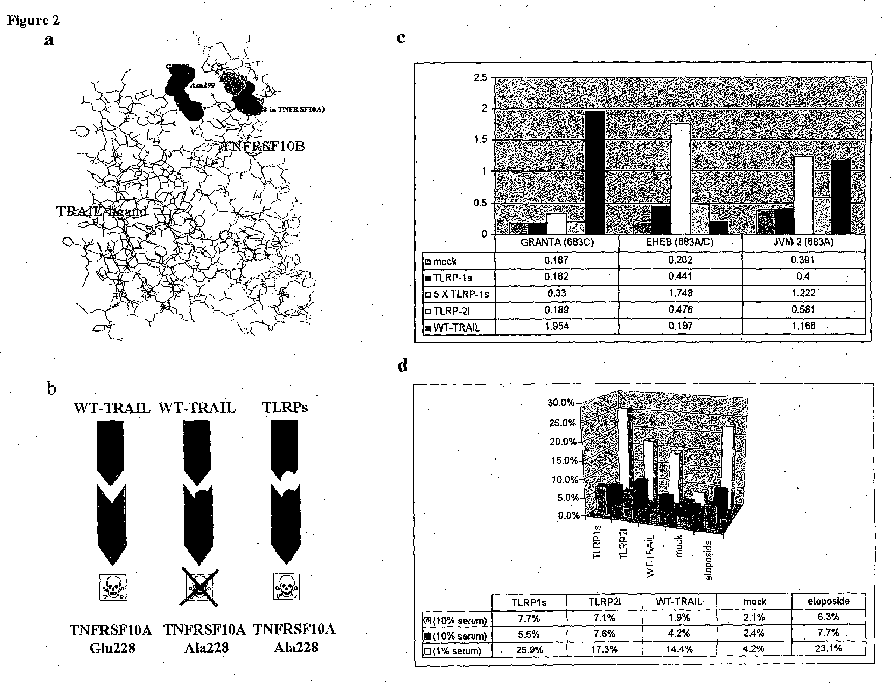

Mutagenized proteins were expressed in *E.coli* using the pCR-T7-CT TOPO expression vector usually for 16 h at 37°C (Figure 2d) and with the pcDNA 3.1/V-5 His mammalian expression vector (Invitrogen, Carlsbad, California) in HeLa cells (Figure 2c). Additionally, the Rapid Translation System 100 *E*. *coli* HY kit (RTS; Roche, Mannheim, Germany) was used with PCR fragments of the genes of interest as a template. For the production of RTS compatible His-tagged PCR-fragments the Rapid Translation System RTS *E*. *coli* Linear Template Generation Set, His-tag (Roche, Mannheim, Germany) was used according to the manufacturers recommendations (Figure 3). Primer sequences and PCR conditions are given below. The HeLa expressed TLRPs were purified using the Probond Purification System (Invitrogen, Carlsbad, California) whereas the RTS derived TLRPs was applied directly onto the cell without prior purification. An aliquot of the TLRPs were separated via PAGE and visualized using an Anti-HIS antibody (Invitrogen, Carlsbad, California) for each experiment.

### (C) Site-directed mutagenesis

Site directed mutagenesis of the TRAIL fragments was performed using the QuikChange site directed mutagenesis kit (Stratagene, La Jolla, California). The sequences of the mutation primers with the resulting amino acid exchanges in the resulting peptides are indicated below.

TLMP1f:5'-CGATTTCAGGAGGAAATAAAAGAAGAAACAAAGAACGACAAACAAATGG-3'

Asn199Glu
TLMP1rev:5'-TGAAATCGCCATTTGTTTGTCGTTCTTTGTTTCTTCTTTTATTTCCTCC-
3'

TLMP2f:5'- CGATTTCAGGAGGAAATAAAAGAAAGAACAAAGAACGACAAACAAATGG-3'
Asn199Arg
TLMP2rev:5'-TGAAATCGCCATTTGTTTGTCGTTCTTTGTTCTTTCTTTTATTTCCTCC-
3'

TLMP3f:5'-CGATTTCAGGAGGAAATAAAAGAATATACAAAGAACGACAAACAAATGG-3'
Asn199Tyr
TLMP3rev:5'-TGAAATCGCCATTTGTTTGTCGTTCTTTGTATATTCTTTTATTTCCTCC-
3'

TLMP4f:5′-CGATTTCAGGAGGAAATAAAAGAACAAACAAAGAACGACAAACAAATGG-3′
Asn199Gln
TLMP4rev:5′-TGAAATCGCCATTTGTTTGTCGTTCTTTGTTTGTTCTTTTATTTCCTCC-
3′

TLMP5f:5'-CGATTTCAGGAGGAAATAAAAGAACTTACAAAGAACGACAAACAAATGG-3'
Asn199Leu
TLMP5rev:5'-TGAAATCGCCATTTGTTTGTCGTTCTTTGTAAGTTCTTTTATTTCCTCC-
3'

TLMP6f:5'-CGATTTCAGGAGGAAATAAAAGAATTCACAAAGAACGACAAACAAATGG-3'
Asn199Phe
TLMP6rev:5'-TGAAATCGCCATTTGTTTGTCGTTCTTTGTGAATTCTTTTATTTCCTCC-
3'

TLMP7f:5'-CGATTTCAGGAGGAAATAAAAGAAGAATATAAGAACGACAAACAAATGG-3'
Asn199Glu; THR200TYR
TLMP7rev:5'-TGAAATCGCCATTTGTTTGTCGTTCTTATATTCTTCTTTTATTTCCTCC-
3'

TLMP8f:5'-CGATTTCAGGAGGAAATAAAAGAAAGATATAAGAACGACAAACAAATGG-3'

Asn199Arg; Thr200Tyr
TLMP8rev:5'-TGAAATCGCCATTTGTTTGTCGTTCTTATATCTTTCTTTTATTTCCTCC-
3'

TLMP9f:5'-CGATTTCAGGAGGAAATAAAAGAAAACGAGAAGAACGACAAACAAATGG-3'
Thr200Glu
TLMP9rev:5'-TGAAATCGCCATTTGTTTGTCGTTCTTCTCGTTTTCTTTTATTTCCTCC-
3'

TLMP10f:5'-CGATTTCAGGAGGAAATAAAAGAAAACTATAAGAACGACAAACAAATGG-3'
Thr200Tyr
TLMP10rev:5'-GAAATCGCCATTTGTTTGTCGTTCTTATAGTTTTCTTTTATTTCCTCC-
3'

### (D) Amplification of TRAIL and TNFRSF10A DNA/cDNA

Genomic DNA- and cDNA-Fragments for TNFRSF10A as well as TRAIL from CLL- and MCL patients, cell lines as well as from B-cells derived from healthy donors were amplified using Klentaq cDNA polymerase (BD Biosciences, Bedford, MA, USA) and Eurotaq Polymerase (BioCat, Heidelberg, Germany) with primers as follows:
Primers for TNFRSF10A mutation analysis on cDNA: 
385f: 5'-GAGAGTTGTGTCCACCAGGATCT -3'
3170rev: 5'-TCCTGAATCTTCTCTTTTGCATGT -3'
 PRC conditions: Initial 94°C denaturation step for 3 min, followed by 40 cycles with 94°C for 10 s, 60°C for 20 s, 72°C for 1 min, followed by a final extension step of 5 min at 72°C.
cDNA sequencing primer: 803rev: 5'-ACAACCTGAGCCGATGCAA -3' Primers for TNFRSF10A mutation analysis on DNA: 
TR1-DNA-f2: 5′-TCCATTGCCTGAGAAAAGACAGG-3′
TR1-DNA-rev2: 5′-ACGCCTTCTCAGGGAGATTGG-3′
 Nested PCR:

TR1-DNA-f3: 5'-TACAGGAGTCTCGGGCTGCTGG-3'
TR1-DNA-rev3: 5'-TCCTCTTTCATCCCACCTGG-3'

PRC and nested PCR conditions: Initial 94°C denaturation step for 3 min, followed by 35 cycles with 94°C for 20 s, 56°C for 20 s, 72°C for 30 s, followed by a final extension step of 7 min at 72°C.

TR1-DNA-f3 and TR1-DNA-rev3 were used as sequencing primers in 10 *µ*l reactions.

Primer sequences for the amplification of TRAIL PCR fragments for cloning into pcDNA 3.1/V-5 His or PCRT7 TOPO vector for protein expression:
TRAIL:
Full length amplicons:

TL1pCRT7f: 5'-ATCATGGCTATGATGGAGGTCCAG -3'
TL1pCRT7rev: 5'-ACTGGCTTCATGGTCCATGTCTATC -3'

PRC conditions: Initial 95°C denaturation step for 3 min, followed by 35 cycles with 94°C for 20 s, 58°C for 30 s, 72°C for 90 s, followed by a final extension step of 5 min at 72°C.

TLspCRT7f: 5'-ACAATGTCCAAGAATGAAAAGGCTCTGG-3'
TLspCRT7rev: 5'-ACTTTTCATCAACAATATAGGGTCAG-3'

PRC conditions: Initial 94°C denaturation step for 2 min, followed by 30 cycles with 94°C for 5 s, 60°C for 10 s, 72°C for 20 s, followed by a final extension step of 2 min at 72°C.

Primer sequences for the amplification of PCR fragments used in the RTS in vitro translation system:

TRAIL
TR1-RTS-c-term,f:  5'-CTTTAAGAAGGAGATATACCATGGCGCCACCACCAGCTAG-
3'
TR1-RTS-c-term,rev:5'-
TGATGATGAGAACCCCCCCCTCCAAGGACACGGCAGAGCCTGTG-3'

TLRP-1s
TLs-RTS-c-term-f:5'-
CTTTAAGAAGGAGATATACCATGTCCAAGAATGAAAAGGCTCTGG-3'
TLs-RTS-c-term-f:5'-
TGATGATGAGAACCCCCCCCACTTTTCATCAACAATATAGGGTCAGG-3'

20 ng of mutagenized TLRP-clone-DNA were used as template in the PCR reactions.

PRC conditions: Initial 95°C denaturation step for 3 min, followed by 30 cycles with 94°C for 10 s, 58°C for 20 s, 72°C for 50 s, followed by a final extension step of 3 min at 72°C. The second that fuses the T7-promotor, T7-terminator and c-terminal his-tag sequences to the PCR template was performed with an annealing temperature of 54°C.

PCR reactions were performed in 50 *µ*l reactions using GeneAmp PCR System 9700 (Applied Biosystems, Foster City, CA) and Klentaq cDNA polymerase (BD Biosciences, Bedford, MA).

### (E) Mutation analysis

The nucleotide sequences of the *TRAIL and TRAIL-R* cDNA and genomic DNA were determined by cycle sequencing with the Big Dye terminator chemistry (Applied Biosystems, Foster City, CA) followed by electrophoresis on a Perkin-Elmer ABI-377 automated sequencer. Clones were sequenced using the standard M13 vector-primers and gene specific primers. 683A/C variants could clearly be detected as a double peak in nucleotide sequence.

### (F) RNA and DNA Preparation

Total RNA and genomic DNA were isolated from the cell lines Granta-519 (DSMZ No: ACC 342), EHEB (DSMZ No: ACC67), and JVM-2 (DSMZ No: ACC12) (Jadayel et *al., Leukemia* **1,** 64-72 (1997); Saltman et *al., Leuk. Res.* **14,** 381-387 (1990); Melo et *al., Int. J. Cancer* **38,** 531-538 (1986)) as well as from mononuclear cell preparations of CLL patients and of control persons (obtained after ficoll density gradient centrifugation) using Trizol reagent (Gibco BRL) according to the manufacturer's protocols. For PCR-analysis of DNA from sputum of heterozygous controls, freshly collected sputum was heated at 100°C for 10 min. A 2 *µ*l aliquot was directly applied to the PCR reaction without further treatment.

### (G) Immunocytochemistry

CLL- and cell line cells were identified using monoclonal mouse anti-human CD19, Clone HD37 (DakoCytomation, Glostrup, Denmark). Antibody was applied to paraformaldehyde-fixed cells and stained with an anti-mouse-cy3 secondary antibody (Dianova, Hamburg, Germany). Experiments were evaluated using a fluorescence microscope (Axioplan, Zeiss, Jena, Germany) and documented using a CCD camera (Photometrics, Huntington Beach, USA).

### Example 2: Apoptosis assays of different TRAIL treated leukaemic cell lines

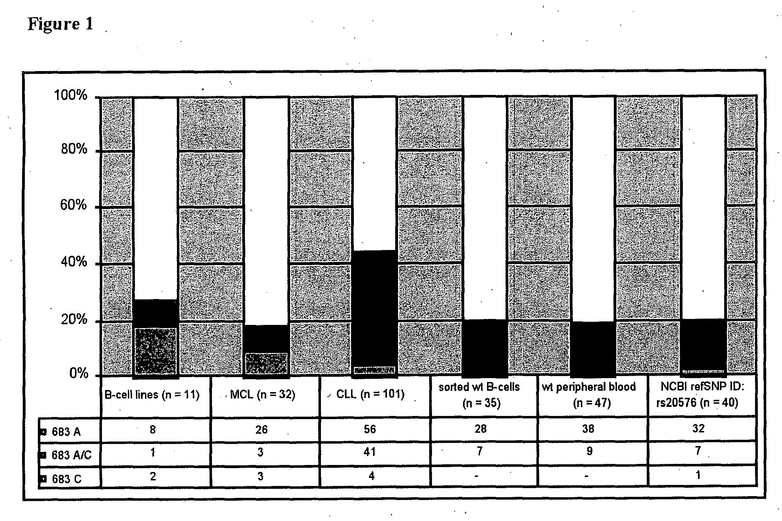

Based on the model that an obstructed TRAIL/TNFRSF10A complex formation is the reason for the resistance of CLL cells against TRAIL induced apoptosis, mutation analysis was carried out on the corresponding gene segment of TNFRSF10A on cDNA or DNA derived from peripheral blood of a series of 101 CLL patients, 32 MCL patients, sorted B-cells from 35 healthy individuals and peripheral blood (PB) samples from 47 healthy individuals. In addition, cDNAs from 11 different cell lines of the B-cell lineage we analyzed. An A→C nucleotide exchange at position 683 in exon 5 of TNFRSF10A resulting in the amino-acid substitution Glu228Ala was detected in more than 40% of the CLL samples. This nucleotide exchange was reported as a SNP in the NCBI database (0.900 A; 0.100 C; Reference SNP: refSNP ID: rs20576). Overall, a 4-fold increased homozygous 683C allele frequency for CLL patients was found, 10-fold for MCL patients and 18-fold for the examined B-cell lines as compared to the NCBI data. In 82 control samples, sorted B-cells and PB samples of healthy donors, no homozygous 683C expression was found at all. 683A/C heterozygosity is found increased more than twofold in CLL samples as compared to B-cell and PB controls and NCBI-SNP database annotation (Figure 1). Logistic regression analysis revealed an estimated 2.96-fold increased risk to develop CLL for heterozygous individuals expressing 683A/C variants (*P* = 0.001), and an estimated 10.59-fold increased risk for individuals exclusively expressing the 683C variant compared to individuals exclusively expressing 683A (*P* = 0.04).

### Example 3: Determination of the origin of the TRAIL polymorphism

In order to determine, whether the polymorphism is of germ-line or somatic origin, the corresponding PCR-fragment of the gene from DNA derived from sputum of 6 heterozygous controls was sequenced. Since both alleles were detected in all of these samples, the polymorphism is present in the germ-line.

### Example 4: Analysis of the functional consequences of the TRAIL amino acid substitution of Glu228Ala

The Glu228Ala substitution resides within the extra-cellular cysteine-rich domain of TNFRSF10A. In the highly homologous TNFRSF10B protein, whose crystal structure in the TNFRSF10B/TRAIL complex has already been resolved, the Glu228 corresponding glutamic acid Glu124 is in close vicinity to TRAIL during TRAIL/TNFRSF10A complex formation and induction of apoptosis (Cha et *al., Immunity* **11**(2), 253-61 (1999); Hymowitz *et al., Mol Cell.* **4**(4) 563-71 (1999) (Figure 2a). The A→C nucleotide exchange on position 683 of TNFRSF10A-sequence leads to the replacement of the negatively charged, large amino acid glutamate by the uncharged, small amino acid alanine, within a highly sensitive region of TRAIL/TNFRSF10A complex formation.

In order to elucidate functional consequences of the 683A/C sequence variants, cell death detection assays on three B-cell lines treated with TRAIL (100 ng/ml) differing in their TNFRSF10A 683 status (EHEB, JVM-2 and GRANTA-519) were carried out. GRANTA-519 exclusively expresses the TNFRSF10A 683A variant; EHEB displays TNFRSF10A 683A/C heterozygosity, whereas JVM-2 is homozygous for TNFRSF10A 683C. In initial pilot cell death assays, EHEB and JVM-2 exhibited a reduced sensitivity to TRAIL induced cell death, compared to GRANTA-519 cells, when exposed to TRAIL (data not shown). This is in line with the model of an obstructed TRAIL/TNFRSF10A interaction.

### Example 5: Mutagenized TRAIL proteins a rcapable of re-inducing the impaired TRAIL mediated apoptosis in patients with TNFRSF10A Ala228 expressing tumor-cells

To test this model, altered TRAIL cDNAs differing in nucleotide sequence at the critical TRAIL-TNFRSF10A interaction domain were designed. The differences caused amino acid alterations in expressed TRAIL peptides around the putative TRAIL/TRAIL-receptor 1 interaction site. The aim was to produce recombinant TRAIL peptides fitting to the rare TNFRSF10A Ala228 variant and capable to restore the induction of apoptosis in these cells (Figure 2b). Site directed mutagenesis was used to produce modified TRAIL cDNAs. These were cloned into yeast expression vectors, creating full-length and truncated 261 bp variants. The PCR fragments contained the nucleotide sequences coding for TNFRSF10A Glu228 corresponding interaction site namely amino acid residues 198-200 of the TRAIL-ligand protein. Additionally, a mammalian expression system and an in vitro translation system were used for expression of the TRAIL protein derivatives *(TRAIL-ligand recombinant proteins,* TLRPs). TLRP expression was assessed by detection of the fused poly histidine-tag with an anti-histidine-antibody in western blot experiments. Mutagenized TLRPs were purified and applied to the two cell lines GRANTA-519 and JVM-2 expressing TNFRSF10A Glu228 and Ala228, respectively, in order to test their capability to induce apoptosis as compared to WT-TRAIL. A photometric immunoassay was employed for the quantitative *in vitro* determination of cytoplasmic histone-associated DNA fragments after induced cell death (data not shown). Based on the results of these pilot experiments, the two mutagenized TRAIL proteins that gave the best results were chosen for further exploration. TLRP-1s is a fragment of the TRAIL protein with Asn199Glu, and TLRP-21 is a full size TRAIL protein with Asn199Arg. Application of TLRP-1s on the three cell lines resulted in cellular responses opposite to the effects obtained by WT-TRAIL treatment: Whereas GRANTA-519 displayed a poor response upon application of TLRP-1s, JVM-2 and EHEB exhibited an increased rate of apoptosis applying TLRP-1s (Figure 2c). In subsequent experiments, a carboxyfluorescein (FAM) labeled caspase-8 inhibitor was used to verify TRAIL/TLRP induced caspase-8 activation in Ala228 homozygous JVM-2 cells. In a set of independent experiments, a clearly enhanced percentage of caspase-8 positive cells following the application of TLRP-1s and TLRP-21 on cell line JVM-2 in comparison to WT-TRAIL was found (Figure 2*d*).

### Example 6: Determination of the therapeutic potential of the mutagenized TRAIL proteins

For determination of the therapeutic potential of the TLRPs not only on cell lines but also on patients, the caspase-8 apoptosis assay was used on B-cells of CLL patients homozygous for Ala228 or Glu228. Lymphocytes were isolated from patients and PCR-derived, in vitro expressed TLRP-1s and WT-TRAIL were applied. After caspase-8 detection assay, cells were fixed on a glass slide and CD19 stained to identify the B-cells. 200-400 B-cells were scored for each experiment to determine TRAIL/TLRP-1s induced apoptosis rates. Application of TLRP-1s onto the CLL-cells of a TNFRSF10A Ala228 homozygous patient resulted in an about 6-fold increased caspase-8 activation as compared to WT-TRAIL. In contrast, TLRP-1s induced no apoptosis in TNFRSF10A Glu228 homozygous, cells whereas WT-TRAIL did (Figure 3). This is in line with the previous findings on the cell lines.

### Conclusions

The above results suggest that the TNFRSF10A Glu228Ala variant is involved in the pathomechanism of a subset of CLL patients. The amino acid substitution very likely leads to a substantial change in the structure of the extra-cellular cysteine-rich domain of TNFRSF10A and thereby to an insufficient interaction of TRAIL during TRAIL/TNFRSF10A complex formation. The consequence is an obstructed induction of caspase-8 dependent apoptosis resulting in a longer survival rate of tumor cells. Since the TNFRSF10A 683A/C heterozygosity occurs in about 20% of healthy individuals, genetic factors different from TRAIL/TRAIL-R dependent apoptosis induction interact with this mutation, increasing the risk of developing disease. The findings of the present invention suggest that individuals homo- or heterozygous for the TNFRSF10A Glu228Ala variant exhibit an 11- and 3-fold enhanced risk, respectively, and that screening for 683A→C nucleotide exchanges may play an important role in diagnosis and/or treatment of this malignancy. Furthermore, the finding of a 10-fold increase of 683C-homozygosity in MCL-patients as compared to healthy individuals suggests a possible role for this variant in the pathogenesis of this disease. Moreover, the generation of mutagenized TRAIL proteins capable of re-inducing the impaired TRAIL mediated apoptosis in patients with TNFRSF10A Ala228 expressing tumor-cells bears the possibility for the development of highly specific drugs.

## Claims

1. A method for diagnosing a subtype of a cell proliferative disorder which is **characterized by** resistance to TRAIL induced apoptosis or a predisposition for such disorder, comprising determining the biological activity and/or level of a TRAIL receptor in a sample from a patient wherein a reduced or eliminated biological activity or level of said TRAIL receptor is indicative of such disorder or predisposition.

2. The method of claim 1, wherein said receptor is TNFRSF10A or TNFRSF10B.

3. The method of claim 2, wherein the TNFRSF10A is **characterized by** a variation within its extracellular domain resulting in a reduced TRAIL binding.

4. The method of claim 3, wherein said variation polymorphism is Glu228Ala.

5. The method of any one of claims 1 to 4, wherein said cell proliferative disorder is CLL, MCL or prostate carcinoma.

6. A polynucleotide encoding TNFRSF10A which is **characterized by** a polymorphism within its extracellular domain resulting in a reduced TRAIL binding.

7. The polynucleotide of claim 6, wherein said polymorphism is a mutation being A683C.

8. A mutated TNFRSF10A encoded by the polynucleotide of claim 6 or 7 or a fragment thereof.

9. A polynucleotide encoding a modified TRAIL which is capable of binding to a mutated TRAIL receptor as defined in any one of claims 1 to 4 such that apoptosis is induced.

10. The polynucleotide of claim 9, wherein the amino acid residue Asn at position 199 of the modified TRAIL is substituted by a different amino acid residue.

11. The polynucleotide of claim 10, wherein the amino acid substitution is Asn199Glu or Asn199Arg.

12. A modified TRAIL encoded by the polynucleotide of any one of claims 9 to 11 or a fragment thereof.

13. An expression vector containing a polynucleotide of claim 6, 7, 9, 10 or 11.

14. A host cell containing the expression vector of claim 13.

15. A method of identifying an agonist/activator of the TRAIL receptor as defined in any one of claims 1 to 4, comprising the following steps:
(a) preparing a candidate compound;
(b) contacting a cell which expresses said TRAIL receptor on its surface with said candidate compound; and
(c) determining whether said candidate compound activates said TRAIL receptor.

16. The method of claim 15, wherein said candidate compound is a modified TRAIL.

17. The method of claim 16, wherein said modified TRAIL contains a mutation within the TRAIL/TRAIL-receptor interaction site.

18. A pharmaceutical composition containing a polynucleotide of any one of claims 9 to 11, an expression vector of claim 13, a modified TRAIL of claim 12 or an agonist/activator identified by the method of any one of claims 15 to 17 and a pharmaceutically acceptable carrier.

19. A method for the production of a pharmaceutical composition comprising the method of 15 and furthermore mixing the agonist/activator with a pharmaceutically acceptable carrier.

20. Use of a polynucleotide of any one of claims 9 to 11, an expression vector of claim 13, a modified TRAIL of claim 12 or an agonist/activator identified by the method of any one of claims 15 to 17 for the preparation of a pharmaceutical composition for reconstituting TRAIL induced apoptosis in TRAIL insensitive cells.

21. Use according to claim 20 for the preparation of a pharmaceutical composition for treating CLL, MCL or prostate carcinoma.
